(19) Europäisches Patentamt
European Patent Office
Office européen des brevets

(11) Numéro de publication : **0 446 131 B1**

(12) # FASCICULE DE BREVET EUROPEEN

(45) Date de publication du fascicule du brevet : **04.05.94 Bulletin 94/18**

(51) Int. Cl.$^5$ : **A61K 7/13**

(21) Numéro de dépôt : **91400617.6**

(22) Date de dépôt : **06.03.91**

(54) **Procédés de teinture des fibres kératiniques avec des monohydroxyindoles substitués, compositions et dispositifs de mise en oeuvre.**

(30) Priorité : **08.03.90 FR 9002976**

(43) Date de publication de la demande : **11.09.91 Bulletin 91/37**

(45) Mention de la délivrance du brevet : **04.05.94 Bulletin 94/18**

(84) Etats contractants désignés : **AT BE CH DE DK ES FR GB GR IT LI NL SE**

(56) Documents cités :
**EP-A- 0 271 186**
**EP-A- 0 348 280**
**FR-A- 2 626 173**
**US-A- 4 013 404**

(73) Titulaire : **L'OREAL**
**14, Rue Royale**
**F-75008 Paris (FR)**

(72) Inventeur : **Junino, Alex**
**16, rue Docteur Bergonié**
**F-93180 Livry-Gargan (FR)**
Inventeur : **Richard, Hervé**
**48, rue de l'Ermitage**
**F-75020 Paris (FR)**
Inventeur : **Vandenbossche, Jean-Jacques**
**37, avenue Berlioz**
**F-93270 Sevran (FR)**

(74) Mandataire : **Casalonga, Axel et al**
**BUREAU D.A. CASALONGA - JOSSE**
**Morassistrasse 8**
**D-80469 München (DE)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention est relative à de nouveaux procédés de teinture des fibres kératiniques et en particulier des fibres kératiniques humaines, telles que les cheveux, en mettant en oeuvre un monohydroxyindole substitué, ainsi qu'aux compositions tinctoriales et aux dispositifs permettant de mettre en oeuvre ces procédés.

Les colorants de la famille des indoles et en particulier le 5,6-dihydroxyindole ainsi que ses dérivés, sont bien connus pour leur utilisation en teinture des fibres kératiniques et notamment des cheveux humains.

Les brevets français FR-A-1 133 594, 1 166 172 et 2 390 158 proposent des procédés de teinture à l'aide du 5,6-dihydroxyindole, en mettant en oeuvre des cations métalliques jouant le rôle de promoteur de la mélanogénèse.

La demande de brevet EP 0 348 280 décrit un procédé de teinture à base de dérivés d'indole et essentiellement le 5,6-dihydroxyindole, en présence d'un sel de terre rare, en tant qu'agent oxydant.

Le 5,6-dihydroxyindole conduit en particulier à des teintes noires ou plus ou moins grises.

On a également décrit dans les FR-A-2 593 061, 2 593 062 et 2 594 331, différents procédés de teinture mettant en oeuvre des dérivés d'indole, en association avec des systèmes oxydants, tels que des anions minéraux, comme par exemple l'iodure ou des anions métalliques, tels que le permanganate ou le bichromate.

Dans la demande de brevet FR 2 626 173, est décrit un procédé de teinture mettant en oeuvre des dérivés d'indole associés à des colorants quinoniques en présence de systèmes oxydants identiques à ceux cités précédemment.

La demanderesse vient de découvrir qu'une classe particulière de monohydroxyindoles substitués sur le noyau aromatique permettait, lorsque la coloration est développée par des agents oxydants, d'obtenir des nuances claires à reflets chauds ou cendrés particulièrement intéressantes.

L'invention a donc pour objet un procédé de teinture des fibres kératiniques et en particulier des fibres kératiniques humaines, en mettant en oeuvre des monohydroxyindoles substitués en association avec un système oxydant.

Un autre objet de l'invention est constitué par les compositions tinctoriales mises en oeuvre au cours de ce procédé, ainsi qu'au dispositif destiné à être utilisé dans la mise en oeuvre de ce procédé.

D'autres objets de l'invention apparaîtront à la lecture de la description et des exemples qui suivent.

Le procédé de teinture des fibres kératiniques et en particulier des fibres kératiniques humaines, telles que les cheveux, conforme à l'invention, est essentiellement caractérisé par le fait que l'on applique sur ces fibres une composition (A) contenant, dans un milieu approprié pour la teinture, au moins un monohydroxyindole substitué, répondant à la formule :

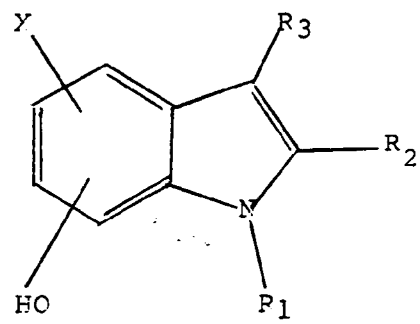

(I)

dans laquelle, le groupement OH substitue l'une quelconque des positions du cycle benzénique, X désigne un groupement alkyle en $C_1$-$C_4$, substitué ou non par un groupement hydroxy, ou désigne un atome d'halogène, ou un groupement

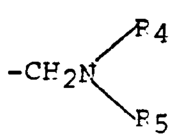

,

dans lequel $R_4$ et $R_5$ désignent,
indépendamment l'un de l'autre, un groupement alkyle en $C_1$-$C_4$, $R_1$ désigne un atome d'hydrogène, un radical

alkyle en $C_1$-$C_4$, $R_2$ et $R_3$, identiques ou différents, désignent un atome d'hydrogène, un radical alkyle en $C_1$-$C_4$, un radical carboxyle ou un radical alcoxycarbonyle; ainsi que leurs sels, la couleur étant révélée à l'aide d'un système oxydant constitué par :

(i) des ions iodure et du peroxyde d'hydrogène, la composition (A) contenant dans ce cas en plus, soit (a) des ions iodure, soit (b) du peroxyde d'hydrogène et l'application de la composition (A) étant précédée ou suivie par l'application d'une composition (B) qui contient, dans un milieu approprié pour la teinture, soit :

(a) du peroxyde d'hydrogène à un pH compris entre 7 et 12 et de préférence entre 2 et 7, lorsque la composition (A) contient des ions iodure,

soit

(b) des ions iodure à un pH compris entre 3 et 11, lorsque la composition (A) contient du peroxyde d'hydrogène;

(ii) des nitrites, l'application de la composition (A) étant suivie par l'application d'une composition aqueuse (B) présentant un pH acide, la composition (A) ou la composition (B) contenant au moins un nitrite;

(iii) des sels métalliques choisis parmi les sels de manganèse, de cuivre, de cobalt et d'argent, ces sels métalliques étant appliqués dans une étape séparée au moyen d'une composition (B) contenant ces sels dans un milieu approprié pour la teinture;

(iv) des sels de terres rares, ces sels de terres rares étant appliqués au moyen d'une composition (B) les contenant dans un milieu approprié pour la teinture, la composition (B) étant appliquée préalablement ou postérieurement à l'application de la composition (A).

Conformément à l'invention, l'application des compositions (A) et (B) est de préférence séparée par un rinçage.

Parmi les dérivés de monohydroxyindoles substitués, répondant à la formule (I), on peut citer :

le 2-carboxy 4-hydroxy 5-méthylindole,
le 4-hydroxy 1,5-diméthylindole,
le 4-hydroxy 5-(diméthylaminométhyl)indole,
le 4-hydroxy 5-méthylindole,
le 2-carboxyméthyl 6-hydroxy 5-tertiobutylindole,
le 4-hydroxy 7-méthylindole,
le 5-hydroxy 4-méthylindole,
le 5-hydroxy 6-méthylindole,
le 6-hydroxy 7-méthylindole,
le 6-hydroxy 7-(diméthylaminométhyl)indole,
le 5-hydroxy 4-(diméthylaminométhyl)indole,
le 7-hydroxy 6-(diméthylaminométhyl)indole,
le 5-hydroxy 3,4-diméthylindole,
le 6-hydroxy 3,7-diméthylindole,
le 4-hydroxy 2,6-diméthylindole,
le 4-hydroxy 6-hydroxyméthylindole,
le 4-hydroxy 6-méthylindole;
et leurs sels.

Les sels sont des sels d'acide cosmétiquement acceptables, et plus particulièrement des chlorhydrates, sulfates, etc...

Selon une première variante du procédé de teinture conforme à l'invention, on applique sur les matières kératiniques une composition (A) contenant, dans un milieu approprié pour la teinture, au moins un composé de formule (I) en association avec des ions iodure, l'application de la composition (A) étant précédée ou suivie par l'application d'une composition (B) qui contient, dans un milieu approprié pour la teinture, du peroxyde d'hydrogène.

Ce procédé peut également être mis en oeuvre en appliquant sur les fibres kératiniques au moins une composition (A) contenant, dans un milieu approprié pour la teinture, le composé de formule (I), en association avec du peroxyde d'hydrogène, ayant un pH compris entre 2 et 7, et de préférence entre 3,5 et 7, l'application de la composition (A) étant précédée ou suivie par l'application d'une composition (B) qui contient dans un milieu approprié pour la teinture, des ions iodure.

L'ion iodure dans cette variante du procédé conforme à l'invention, est choisi de préférence parmi les iodures de métaux alcalins, alcalino-terreux ou d'ammonium et plus particulièrement est constitué par l'iodure de potassium.

Les ions iodure sont présents dans les compositions (A) ou (B) dans des proportions comprises généralement entre 0,007 et 4% en poids exprimé en ions $I^-$ et de préférence entre 0,08 et 1,5% en poids par rapport au poids total de la composition (A) ou (B).

Selon une seconde variante de l'invention, on peut mettre en oeuvre le procédé en utilisant comme agent oxydant pour révéler la coloration, un nitrite. Les nitrites plus particulièrement utilisables, conformément à l'invention, sont :

- des nitrites de métaux alcalins, alcalino-terreux ou d'ammonium ou de tout autre cation cosmétiquement acceptable, lorsqu'il est utilisé pour la teinture des cheveux humains vivants;
- des dérivés organiques des nitrites, tels que par exemple le nitrite d'amyle;
- des vecteurs de nitrites, c'est-à-dire des composés qui par transformation génèrent un nitrite du type défini ci-dessus.

Les nitrites particulièrement préférés sont les nitrites de sodium, de potassium ou d'ammonium.

Cette variante du procédé est mise en oeuvre en appliquant sur les matières kératiniques la composition (A) à base du composé de formule (I) définie ci-dessus, puis celle d'une composition aqueuse acide (B), la composition (A) ou (B) contenant au moins un nitrite.

Les nitrites sont généralement utilisés dans des proportions comprises entre 0,02 et 1 mole/litre.

Selon une troisième variante de l'invention, on met en oeuvre des catalyseurs d'oxydation choisis parmi les sels métalliques, tels que les sels de manganèse, de cobalt, de cuivre et d'argent. A titre d'exemple, on peut citer le sulfate de manganèse, le lactate de manganèse, le chlorure de cobalt, le chlorure cuivrique, le nitrate d'argent ammoniacal. Les sels préférés sont les sels de cuivre. Ces sels sont utilisés dans des proportions de 0,01 à 2% exprimées en ion métallique.

Selon cette variante, on met les fibres kératiniques et en particulier les cheveux humains, en contact avec une composition (B) contenant dans un milieu approprié pour la teinture, le sel métallique avant ou après l'application de la composition (A) contenant le composé de formule (I) et on rince de préférence entre les deux étapes.

La forme de réalisation préférée consiste à appliquer un sel cuivrique dans un premier temps et la composition (A) dans un deuxième temps.

On peut faire suivre cette teinture, après rinçage, de l'application d'une solution de peroxyde d'hydrogène.

Selon une quatrième variante, on utilise des sels de terres rares. Les sels de terres rares utilisables, conformément à l'invention, sont choisis parmi les sels de Lanthanides, et notamment les sels de Cérium $Ce^{3+}$, $Ce^{4+}$, de Lanthane $La^{3+}$, d'Europium $Eu^{2+}$, $Eu^{3+}$, de Gadolinium $Gd^{3+}$, d'Ytterbium $Yb^{2+}$, $Yb^{3+}$, de Dysprosium $Dy^{3+}$. Les sels préférés sont en particulier les sulfates, chlorures ou nitrates.

Ces sels de terres rares sont présents de préférence dans des proportions comprises entre 0,1 et 8% en poids par rapport au poids total de la composition.

On utilise de préférence les sels de Cérium $Ce^{3+}$ et $Ce^{4+}$ sous la forme de sulfates et de chlorures.

Lorsque des compositions à base de peroxyde d'hydrogène sont utilisées, la teneur en peroxyde d'hydrogène est généralement comprise entre 1 et 40 volumes et de préférence entre 2 et 10 volumes, et plus particulièrement entre 3 et 10 volumes.

La quantité de mononydroxyindoles substitués répondant à la formule (I) utilisée dans la composition (A), conforme à l'invention, est généralement comprise dans des proportions de 0,01 et 5% en poids par rapport au poids total de la composition, et de préférence entre 0,03 et 2,5% en poids.

Les compositions utilisables, conformément à l'invention, peuvent se présenter sous des formes diverses, telles que sous forme de lotions plus ou moins épaissies, de crèmes, de mousses, de gels. Ces compositions peuvent également être présentées dans des dispositifs à plusieurs compartiments ou kits de teinture contenant les différents composants destinés à être mélangés au moment de l'emploi, ou encore sous la forme d'aérosols.

Les compositions utilisables dans le procédé conforme à l'invention et qui constituent également un objet de l'invention, sont caractérisées par le fait qu'elles contiennent dans un milieu approprié pour la teinture, au moins un dérivé de monohydroxyindole substitué de formule (I), et au moins des ions iodure ou des nitrites, tels que définis ci-dessus.

Les proportions des différents constituants sont telles que définies également ci-dessus.

Le milieu approprié pour la teinture est de préférence un milieu aqueux qui doit être cosmétiquement acceptable lorsque les compositions sont destinées à la teinture des cheveux humains vivants. Ce milieu aqueux peut être constitué par de l'eau ou un mélange eau/solvant (s).

Les solvants sont choisi parmi les solvants organiques et préférentiellement parmi l'alcool éthylique, l'alcool propylique ou isopropylique, l'alcool tertiobutylique, l'éthylèneglycol, les éthers monométhylique, monoéthylique et monobutylique de l'éthylèneglycol, l'acétate du monoéthyléther de l'éthylèneglycol, le propylèneglycol, les monométhyléthers du propylèneglycol et du dipropylèneglycol, le lactate de méthyle.

Les solvants particulièrement préférés sont l'alcool éthylique et le propylèneglycol.

Selon une autre forme de réalisation, le milieu approprié pour la teinture peut être constitué par des solvants anhydres, tels que ceux définis préférentiellement ci-dessus, la composition étant dans ce cas, soit mélangée au moment de l'emploi avec le milieu aqueux, soit appliquée sur les fibres kératiniques mouillées au préalable par une composition aqueuse. On appelle, conformément à l'invention, un milieu solvant anhydre, un milieu contenant moins de 1% d'eau.

Le pH de la composition (A), lorsque le milieu approprié pour la teinture est aqueux, est compris de préférence entre 2 et 7, et en particulier entre 3,5 et 7.

Lorsque le milieu approprié pour la teinture est constitué par un mélange eau/solvant(s), les solvants sont utilisés dans des concentrations comprises entre 0,5 et 75% en poids par rapport au poids total de la composition, et de préférence entre 2 et 50%, et plus particulièrement entre 2 et 20% en poids.

Les compositions conformes à l'invention peuvent contenir tous autres adjuvants habituellement utilisés pour la teinture des fibres kératiniques et en particulier des adjuvants cosmétiquement acceptables, dans la mesure où ces compositions sont appliquées pour la teinture des cheveux humains vivants.

Dans ce dernier cas, les compositions peuvent contenir notamment des amides gras dans des proportions préférentielles de 0,05 à 10% en poids, des agents tensio-actifs anioniques, cationiques, non ioniques, amphotères ou leurs mélanges, présents de préférence dans des proportions comprises entre 0,1 à 50% en poids, des agents épaississants, des parfums, des agents séquestrants, des agents filmogènes, des agents de traitement, des agents dispersants, des agents de conditionnement, des agents conservateurs, des agents opacifiants, des agents de gonflement des fibres kératiniques, etc...

Les épaississants sont choisis parmi des agents habituellement utilisés en cosmétique et plus particulièrement parmi l'alginate de sodium, la gomme arabique, la gomme de guar, les hétérobiopolysaccharides, tels que la gomme de xanthane, les scléroglucanes, les dérivés de cellulose, tels que la méthylcellulose, l'hydroxyéthylcellulose, l'hydroxypropylméthylcellulose, le sel de sodium de la carboxyméthylcellulose et les polymères d'acide acrylique préférentiellement réticulés.

On peut également utiliser des agents épaississants minéraux, tels que la bentonite. Ces épaississants sont utilisés seuls ou en mélange et sont présents de préférence dans des proportions comprises entre 0,1 et 5% en poids par rapport au poids total de la composition et avantageusement entre 0,5 et 3% en poids.

Les agents alcalinisants utilisables dans les compositions, peuvent être en particulier des amines, telles que les alcanolamines, des alkylamines, des hydroxydes ou carbonates alcalins ou d'ammonium.

Les agents acidifants, utilisables dans ces compositions, peuvent être choisis parmi l'acide lactique, l'acide acétique, l'acide tartrique, l'acide phosphorique, l'acide chlorhydrique, l'acide citrique.

Il est bien entendu possible d'utiliser tout autre agent alcalinisant ou acidifiant acceptable, notamment dans le cas de la teinture des cheveux humains.

Lorsque les compositions sont utilisées sous forme de mousse, elles peuvent être conditionnées sous pression et dans un dispositif aérosol, en présence d'un agent propulseur et d'au moins un générateur de mousse.

Les agents générateurs de mousse peuvent être des polymères moussants anioniques, cationiques, non ioniques, amphotères ou leurs mélanges ou des agents tensio-actifs du type de ceux définis ci-dessus.

L'invention a également pour objet un agent de coloration des fibres kératiniques et en particulier des fibres kératiniques humaines, comportant plusieurs composants, dont l'un des composants est constitué par la composition (A) définie ci-dessus et l'autre composant est constitué par l'une des compositions (B) également définie(s) ci-dessus. Les composants (A) et (B) respectifs étant choisis selon les différentes variantes exposées ci-dessus.

L'invention a également pour objet un dispositif à plusieurs compartiments encore appelé "kit de teinture" ou "nécessaire de teinture", comportant tous les composants destinés à être appliqués pour une même teinture sur les fibres kératiniques, en applications successives avec ou sans prémélange.

De tels dispositifs sont connus en eux-mêmes et peuvent comporter un premier compartiment contenant la composition (A), comprenant le dérivé de monohydroxyindole substitué de formule (I) dans un milieu approprié pour la teinture et dans un second compartiment, une composition (B) définie ci-dessus.

Lorsque le milieu contenant le dérivé de monohydroxyindole substitué est anhydre, on peut prévoir un troisième compartiment contenant un milieu aqueux approprié pour la teinture destiné à être mélangé avant l'emploi avec la composition de ce premier compartiment.

Les dispositifs à plusieurs compartiments utilisables conformément à l'invention, peuvent être équipés de moyens de mélange au moment de l'emploi et leur contenu peut être conditionné sous atmosphère inerte.

Le procédé et les compositions utilisés, conformément à l'invention, peuvent être mis en oeuvre pour teindre les cheveux naturels ou déjà teints, permanentés ou non, défrisés ou non, ou les cheveux fortement ou légèrement décolorés, éventuellement permanentés. Il est également possible de les utiliser pour la teinture de la fourrure ou de la laine.

Les exemples qui suivent sont destinés à illustrer l'invention sans pour autant présenter un caractère limitatif.

EXEMPLES DE PREPARATION

EXEMPLE 1

PREPARATION DU 1,5-DIMETHYL 4-HYDROXYINDOLE.

a) Préparation du 4-benzyloxy 1,5 diméthylindole.

On introduit successivement le 4-benzyloxy 5-méthylindole obtenu selon F. TROXLER et al., Helvetica Chimica Acta, 51, 1203 (1968) (2,5 g, 0,0105 mole), de la soude à 50% (12 ml), du toluène (7 ml) et de l'hydrogénosulfate de tétrabutylammonium (280 mg). On chauffe à 60°C et introduit le diméthylsulfate (1,2 ml). On laisse à 60°C pendant 30 minutes, dilue à l'eau et sépare les deux phases. On lave la phase organique à l'eau, la sèche et évapore le solvant. On obtient une huile vert pâle. Après chromatographie sur silice (éluant : $CH_2Cl_2$/heptane 50:50), on obtient une huile incolore de 4-benzyloxy 1,5-diméthylindole (1,9 g, rendement = 72%).

Analyse pour $C_{17}H_{17}NO$

| | C | H | N | O |
|---|---|---|---|---|
| Calculé | 81,24 | 6,82 | 5,57 | 6,37 |
| Trouvé | 81,06 | 6,88 | 5,45 | 6,28 |

b) Préparation du 1,5-diméthyl 4-hydroxyindole.

Un mélange du dérivé précédent (1,8 g, 0,0072 mole), de cyclohexène (2,5 ml), d'éthanol absolu (15 ml) et de palladium sur charbon (0,4 g), est porté au reflux pendant 3 heures. On filtre à chaud et évapore le solvant. On obtient après passage sur colonne de silice (éluant $CH_2Cl_2$), une poudre blanc cassé de 1,5-diméthyl 4-hydroxyindole (0,5 g, rendement = 44%, Pf = 68°C).

Analyse pour $C_{10}H_{11}NO$

| | C | H | N | O |
|---|---|---|---|---|
| Calculé | 74,51 | 6,88 | 8,69 | 9,92 |
| Trouvé | 74,48 | 6,90 | 8,72 | 10,10 |

EXEMPLE 2

PREPARATION DU 2-CARBOXY 4-HYDROXY 5-METHYLINDOLE.

On introduit successivement le 4-benzyloxy 2-carboxy 5-méthylindole obtenu selon F. TROXLER et al., Helvetica Chimica Acta, 51, 1203 (1968) (0,8 g, 2,84 $10^{-3}$ mole), du cyclohexène (1,5 ml), de l'éthanol absolu (8 ml) et du palladium sur charbon (0,2 g). On porte au reflux pendant 2 heures. On filtre à chaud, rince copieusement au méthanol chaud et évapore le solvant. On recristallise dans le méthanol pour obtenir une poudre blanc cassé de 2-carboxy 4-hydroxy 5-méthylindole.
(0,5 g, rendement de 61% et un point de fusion de 62°C).

EXEMPLES D'APPLICATION

EXEMPLE 1

- 4-hydroxy 5-méthylindole 0,5 g
- Iodure de potassium 1,0 g
- Alcool éthylique 15,0 g
- Eau qsp 100,0 g

Cette composition est appliquée pendant 5 minutes sur des cheveux gris à 95% de blancs.

On applique alors, après essorage intermédiaire, sur ces cheveux, pendant 5 minutes, un lait oxydant à pH 3 titrant 12,5 volumes d'eau oxygénée. On rince et on sèche. Les cheveux sont alors colorés dans une nuance cendrée.

EXEMPLE 2

- 4-hydroxy 5-(diméthylaminométhyl)indole 0,5 g
- Iodure de potassium 1,0 g
- Alcool éthylique 15,0 g
- Eau qsp 100,0 g

Cette composition est appliquée pendant 5 minutes sur des cheveux gris à 95% de blancs.

On applique alors, après essorage intermédiaire, sur ces cheveux, pendant 5 minutes, un lait oxydant à pH 3 titrant 12,5 volumes d'eau oxygénée. Après shampooing et rinçage à l'eau, puis séchage, on obtient une coloration beige clair légèrement verdâtre.

EXEMPLE 3

- 1,5-diméthyl 4-hydroxyindole 0,5 g
- Iodure de potassium 1,0 g
- Alcool éthylique 15,0 g
- Eau qsp 100,0 g

Cette composition est appliquée pendant 5 minutes sur des cheveux décolorés.

On applique alors, après essorage intermédiaire, sur ces cheveux, pendant 5 minutes, un lait oxydant à pH 3 titrant 12,5 volumes d'eau oxygénée. On rince et on sèche. Les cheveux sont alors colorés beige cendré à reflets rouges.

EXEMPLE 4

- 2-carboxy 4-hydroxy 5-méthylindole 0,5 g
- Iodure de potassium 1,0 g
- Alcool éthylique 15,0 g
- Eau qsp 100,0 g

Cette composition est appliquée pendant 5 minutes sur des cheveux décolorés.

On applique alors, après essorage intermédiaire, sur ces cheveux, pendant 5 minutes, un lait oxydant à pH 3 titrant 12,5 volumes d'eau oxygénée. On rince et on sèche. Les cheveux sont alors colorés lilas cendré.

EXEMPLE 5

COMPOSITION (A)

- 6-hydroxy 5-méthylindole 0,5 g
- Alcool éthylique à 96° 6,5 g
- Propylèneglycol 2,0 g
- Nonylphénol oxyéthyléné à 9 moles d'oxyde d'éthylène 4,0 g
- Acide citrique qs pH=6,6
- Eau qsp 100,0 g

COMPOSITION (B)

- Nitrite de sodium 2,0 g
- Acide chlorhydrique qs pH=3,8
- Eau qsp 100,0 g

La composition (A) est appliquée pendant 20 minutes sur des cheveux gris naturels à 90% de blancs.

On applique alors, après essorage intermédiaire, sur ces cheveux, la composition (B) pendant 5 minutes. On rince et on sèche. Les cheveux sont alors colorés blond très clair naturel dans une nuance cendrée dorée.

EXEMPLE 6

COMPOSITION (A)

- Sulfate de cuivre à 5 molécules d'eau 1,0 g
- Lauryléther sulfate de sodium à 2 moles d'oxyde d'éthylène 15,0 g
- Hydroxyéthylcellulose à 16% 15,0 g
- Monoéthanolamine qs pH=9,5
- Eau qsp 100,0 g

COMPOSITION (B)

- 4-hydroxy 5-méthylindole 1,0 g
- Alcool éthylique à 96° 10,0 g
- Lauryéther sulfate de sodium à 2 moles d'oxyde d'éthylène 15,0 g
- Triéthanolamine qs pH=6,8
- Eau qsp 100,0 g

La composition (A) est appliquée pendant 5 minutes sur des cheveux gris nauturels à 90% de blancs. Après rinçage, on applique, sur ces cheveux, la composition (B) pendant 10 minutes. On rince et on sèche. On obtient des cheveux colorés blond cendré légèrement doré mat.

EXEMPLE 7

COMPOSITION (A)

- Sulfate de cérium 1,0 g
- Eau qsp 100,0 g
- pH spontané = 1,6

COMPOSITION (B)

- 4-hydroxy 5-(diméthylaminométhyl)indole 1,0 g
- Monobutyléther d'éthylèneglycol 10,0 g
- Nonylphénol oxyéthyléné à 9 moles d'oxyde d'éthylène 15,0 g
- Acide citrique qs pH=6,9
- Eau qsp 100,0 g

La composition (A) est appliquée pendant 10 minutes sur les cheveux gris permanentés à 90% de blancs. Après rinçage, on applique, sur ces cheveux, la composition (B) pendant 10 minutes. On rince et on sèche. On obtient des cheveux colorés blond foncé cendré mat.

## Revendications

1. Procédé de teinture des fibres kératiniques, en particulier des fibres kératiniques humaines, caractérisé par le fait que l'on applique sur ces fibres une composition (A) contenant, dans un milieu approprié pour la teinture, au moins un monohydroxyindole substitué, répondant à la formule :

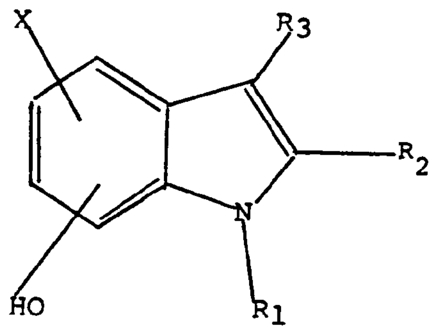

(I)

dans laquelle OH occupe l'une quelconque des positions du cycle benzénique, X désigne un radical alkyle en $C_1$-$C_4$, substitué ou non par un groupement hydroxy, X pouvant également désigner halogène ou un groupement

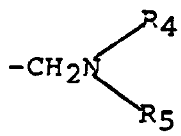

,

dans lequel $R_4$ et $R_5$ désignent un groupement alkyle en $C_1$-$C_4$, $R_1$ désigne un atome d'hydrogène, un radical alkyle en $C_1$-$C_4$, $R_2$ et $R_3$, identiques ou différents, désignent, indépendamment l'un de l'autre, un atome d'hydrogène, un radical alkyle en $C_1$-$C_4$, un radical carboxyle ou un radical alcoxy-carbonyle; ainsi que leurs sels, la couleur étant révélée à l'aide d'un système oxydant constitué par :

(i) des ions iodure et du peroxyde d'hydrogène, la composition (A) contenant dans ce cas en plus, soit (a) des ions iodure, soit (b) du peroxyde d'hydrogène, l'application de la composition (A) étant précédée ou suivie par l'application d'une composition (B) qui contient, dans un milieu approprié pour la teinture, soit :

(a) du peroxyde d'hydrogène à un pH compris entre 2 et 12, lorsque la composition (A) contient des ions iodure, soit

(b) des ions iodure à un pH compris entre 3 et 11, lorsque la composition (A) contient du peroxyde d'hydrogène;

(ii) des nitrites, l'application de la composition (A) étant suivie par l'application d'une composition aqueuse (3) présentant un pH acide, la composition (A) ou la composition (B) contenant au moins un nitrite;

(iii) des sels métalliques choisis parmi les sels de manganèse, de cobalt, de cuivre et d'argent, ces sels métalliques étant appliqués dans une étape séparée au moyen d'une composition (B) contenant ces sels, dans un milieu approprié pour la teinture;

(iv) des sels de terres rares, ces sels de terres rares étant appliqués au moyen d'une composition (B) les contenant dans un milieu approprié pour la teinture, la composition (B) étant appliquée préalablement ou postérieurement à l'application de la composition (A).

2. Procédé selon la revendication 1, caractérisé par le fait que l'application des compositions (A) et (B) est séparée par une étape de rinçage à l'eau.

3. Procédé selon l'une quelconque des revendications 1 ou 2, caractérisé par le fait que les monohydroxyindoles de formule (I) sont choisis parmi :

le 2-carboxy 4-hydroxy 5-méthylindole,
le 4-hydroxy 1,5-diméthylindole,
le 4-hydroxy 5-(diméthylaminométhyl)indole,
le 4-hydroxy 5-méthylindole,
le 2-carboxyméthyl 6-hydroxy 5-tertiobutylindole,
le 4-hydroxy 7-méthylindole,
le 5-hydroxy 4-méthylindole,
le 5-hydroxy 6-méthylindole,
le 6-hydroxy 7-méthylindole,
le 6-hydroxy 7-(diméthylaminométhyl)indole,

le 5-hydroxy 4-(diméthylaminométhyl)indole,
le 7-hydroxy 6-(diméthylaminométhyl)indole,
le 5-hydroxy 3,4-diméthylindole,
le 6-hydroxy 3,7-diméthylindole,
le 4-hydroxy 2,6-diméthylindole,
le 4-hydroxy 6-hydroxyméthylindole,
le 4-hydroxy 6-méthylindole;
et leurs sels.

**4.** Procédé selon l'une quelconque des revendications 1 à 3, caractérisé par le fait que l'on applique sur les matières kératiniques une composition (A) contenant, dans un milieu approprié pour la teinture, au moins un monohydroxyindole substitué, de formule (I), en association avec des ions iodure, l'application de la composition (A) étant précédée ou suivie par l'application d'une composition (B) qui contient, dans un milieu approprié pour la teinture, du peroxyde d'hydrogène.

**5.** Procédé selon l'une quelconque des revendications 1 à 3, caractérisé par le fait que l'on applique sur les fibres kératiniques, au moins une composition (A) contenant, dans un milieu approprié pour la teinture, un monohydroxyindole substitué de formule (I), en association avec du peroxyde d'hydrogène et ayant un pH compris entre 2 et 7, l'application de la composition (A) étant précédée ou suivie par l'application d'une composition (B) qui contient, dans un milieu approprié pour la teinture, des ions iodure.

**6.** Procédé selon l'une quelconque des revendications 4 ou 5, caractérisé par le fait que les ions iodure sont présents dans les compositions (A) ou (B) dans des proportions comprises entre 0,007 et 4% en poids exprimé en ions $I^-$, par rapport au poids total de la composition (A) ou (B).

**7.** Procédé selon l'une quelconque des revendications 1 à 3, caractérisé par le fait que l'on applique sur les matières kératiniques une composition (A) à base du monohydroxyindole substitué de formule (I), que l'on applique ensuite une composition aqueuse acide (B), la composition (A) ou la composition (B) contenant au moins un nitrite choisi parmi les nitrites de métaux alcalins, alcalino-terreux ou d'ammoniun ou de tout autre cation cosmétiquement acceptable, de dérivés organiques et de nitrites et de vecteurs de nitrites générant un nitrite du type défini ci-dessus.

**8.** Procédé selon la revendication 7, caractérisé par le fait que les nitrites sont présents dans des proportions comprises entre 0,02 et 1 mole/ litre.

**9.** Procédé selon l'une quelconque des revendications 1 à 3, caractérisé par le fait que l'on applique sur les fibres kératiniques une composition (A) contenant, dans un milieu approprié pour la teinture, un monohydroxyindole substitué de formule (I) et que l'on applique avant ou après la composition (A), une composition (B) contenant, dans un milieu approprié pour la teinture, un sel métallique de manganèse, de cobalt, de cuivre ou d'argent.

**10.** Procédé selon la revendication 9, caractérisé par le fait que les sels de métaux sont utilisés dans des proportions comprises entre 0,01 et 2% en poids exprimés en ions métalliques par rapport au poids total de la composition.

**11.** Procédé selon l'une quelconque des revendications 1 à 3, caractérisé par le fait que l'on applique une composition (A) contenant, dans un milieu approprié pour la teinture, au moins un monohydroxyindole substitué tel que défini dans l'une quelconque des revendications 1 à 3, et qu'avant ou après cette composition, on applique une composition (B) contenant, dans un milieu approprié pour la teinture, un sel de terres rares choisi parmi les sels de Cérium, de Lanthane, d'Europium, de Gadolinium, d'Ytterbium, de Dysprosium.

**12.** Procédé selon la revendication 11, caractérisé par le fait que les sels de terres rares sont présents dans des proportions comprises entre 0,1 et 8% en poids par rapport au poids total de la composition.

**13.** Procédé selon l'une quelconque des revendications 1 à 12, caractérisé par le fait que lorsqu'on utilise comme moyen oxydant une composition à base de peroxyde d'hydrogène, la teneur en peroxyde d'hydrogène dans la composition est généralement comprise entre 1 et 40 volumes et de préférence entre 2 et 10 volumes.

**14.** Procédé selon l'une quelconque des revendications 1 à 13, caractérisé par le fait que la quantité de monohydroxyindoles substitués de formule (I) utilisée dans la composition (A), est comprise entre 0,01 et 5% en poids par rapport au poids total de la composition, et de préférence entre 0,03 et 2,5% en poids.

**15.** Procédé selon l'une quelconque des revendications 1 à 14, caractérisé par le fait que les compositions (A) et (B) se présentent sous forme de lotions plus ou moins épaissies, de crèmes, de mousses, de gels.

**16.** Procédé selon l'une quelconque des revendications 1 à 15, caractérisé par le fait que le milieu approprié pour la teinture utilisé pour les différentes compositions (A) et/ou (B), est un milieu aqueux constitué par de l'eau ou un mélange d'eau et d'un solvant organique, ou bien est constitué par un milieu solvant anhydre.

**17.** Procédé selon la revendication 16, caractérisé par le fait que les solvants utilisés sont choisis parmi l'alcool éthylique, l'alcool propylique ou isopropylique, l'alcool tertiobulytique, l'éthylène glycol, les éthers monométhylique, monoéthylique et monobutylique de l'éthylèneglycol, l'acétate du monoéthyléther de l'éthylèneglycol, le propylèneglycol, les monométhyléthers du propylèneglycol et du dipropylèneglycol, le lactate de méthyle.

**18.** Composition destinée à être utilisée dans la teinture des fibres kératiniques, caractérisée par le fait qu'elle contient dans un milieu approprié pour la teinture de ces fibres kératiniques, au moins un monohydroxyindole répondant à la formule (I) :

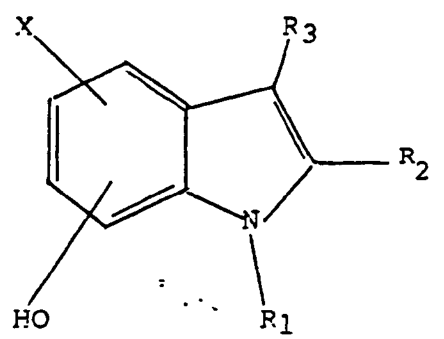

(I)

dans laquelle OH occupe l'une quelconque des positions du cycle benzénique, X désigne un radical alkyle en $C_1$-$C_4$, substitué ou non par un groupement hydroxy, X pouvant également désigner halogène ou un groupement

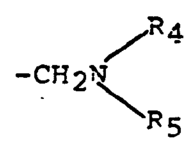

,

dans lequel $R_4$ et $R_5$ désignent,
indépendamment l'un de l'autre, alkyle en $C_1$-$C_4$ ou hydrogène; $R_1$ désigne un atome d'hydrogène ou un radical alkyle en $C_1$-$C_4$, $R_2$ et $R_3$, identiques ou différents, désignent un atome d'hydrogène, un radical alkyle en $C_1$-$C_4$, un radical carboxyle ou un radical alcoxycarbonyle; ainsi que leurs sels, et au moins des ions iodure ou des nitrites.

**19.** Agent de teinture des fibres kératiniques, en particulier des fibres kératiniques humaines, à plusieurs composants, caractérisé par le fait qu'il comprend un premier composant constitué par une composition (A), contenant un monohydroxyindole substitué de formule (I) telle que définie dans une des revendications précédentes, et un second composant constitué par l'une des compositions (B) telle que définie dans une des revendications précédentes.

**20.** Dispositif à plusieurs compartiments ou kit de teinture, comprenant plusieurs compartiments :
a) le premier compartiment contenant une composition (A) contenant un monohydroxyindole substitué de formule (I) telle que définie dans une des revendications précédentes dans un milieu anhydre,

b) un second compartiment contenant une composition (B) choisie parmi :

(i) du péroxyde d'hydrogène dans un milieu acceptable pour la teinture des fibres kératiniques,

(ii) une source d'ions iodure dans un milieu acceptable pour la teinture de ces fibres,

(iii) une source de nitrites dans une composition aqueuse ayant un pH acide,

(iv) des sels métalliques choisis parmi les sels de manganèse, cobalt, cuivre et argent dans un milieu acceptable pour la teinture de ces fibres,

(v) des sels de terres rares dans un milieu acceptable pour la teinture des fibres ; et

c) un troisième compartiment constitué par un milieu aqueux cosmétiquement acceptable, destiné à être mélangé avant l'emploi avec la composition (A) contenue dans le premier compartiment.

## Patentansprüche

1. Verfahren zur Färbung keratinischer Fasern, insbesondere menschlicher keratinischer Fasern, dadurch **gekennzeichnet**, daß man auf diese Fasern eine Zusammensetzung (A) aufbringt, die, in einem zur Färbung geeigneten Milieu, mindestens ein substituiertes Monohydroxyindol der Formel:

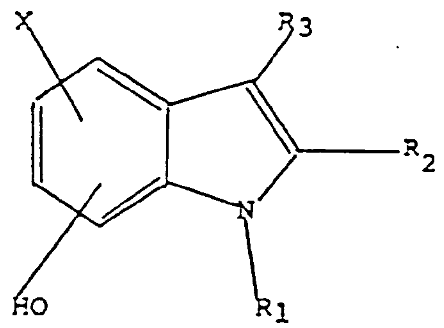

(I)

worin die OH-Gruppe eine jeder Positionen des Benzolringes substituiert, X eine $C_{1-4}$-Alkylgruppe, die durch eine Hydroxygruppe substituiert ist oder nicht, oder ein Halogenatom oder eine Gruppe

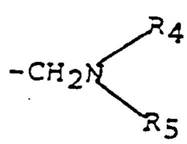

,

worin $R_4$ und $R_5$, unabhängig voneinander eine $C_{1-4}$-Alkylgruppe oder Wasserstoff bedeuten, $R_1$ ein Wasserstoffatom, einen $C_{1-4}$-Alkylrest, $R_2$ und $R_3$, gleich oder verschieden, ein Wasserstoffatom, einen $C_{1-4}$-Alkylrest, einen Carboxyl- oder Alkoxicarbonylrest darstellen, sowie dessen Salze enthält, wobei die Farbe mittels eines oxidierenden Systems entwickelt wird, das dargestellt ist durch:

(i) Jodidionen und Wasserstoffperoxid, wobei die Zusammensetzung (A) in diesem Fall zusätzlich entweder (a) Jodidionen oder (b) Wasserstoffperoxid enthält, und die Aufbringung der Zusammensetzung (A) vor oder nach der Aufbringung einer Zusammensetzung (B) erfolgt, die, in einem zur Färbung geeigneten Milieu, entweder:

(a) Wasserstoffperoxid bei einem pH von 2 bis 12 und vorzugsweise von 2 bis 7, wenn die Zusammensetzung (A) Jodidionen enthält,

oder

(b) Jodidionen bei einem pH von 3 bis 11 enthält, wenn die Zusammensetzung (A) Wasserstoffperoxid enthält;

(ii) durch Nitrite, wobei im Anschluß an die Aufbringung der Zusammensetzung (A) die Aufbringung einer wässrigen Zusammensetzung (B) erfolgt, die einen sauren pH aufweist, wobei die Zusammensetzung (A) oder die Zusammensetzung (B) mindestens ein Nitrit enthalten;

(iii) durch Metallsalze, ausgewählt aus Mangan-, Kupfer-, Kobalt- und Silbersalzen, wobei diese Metallsalze in einer getrennten Stufe mittels einer Zusammensetzung (B) aufgebracht werden, die diese Salze in einem zur Färbung geeigneten Milieu enthält;
(iv) durch Salze der seltenen Erden, wobei diese Salze der seltenen Erden mittels einer sie in einem zur Färbung geeigneten Milieu enthaltenden Zusammensetzung (B) aufgetragen werden, wobei die Zusammensetzung (B) vor oder nach der Aufbringung der Zusammensetzung (A) aufgetragen wird.

2. Verfahren gemäß Anspruch 1,
dadurch **gekennzeichnet**, daß
die Aufbringung der Zusammensetzungen (A) und (B) durch eine Stufe der Spülung mit Wasser getrennt ist.

3. Verfahren gemäß jedem Anspruch 1 oder 2,
dadurch **gekennzeichnet**, daß
die Monohydroxyindole der Formel (I) ausgewählt sind aus:
2-Carboxy-4-hydroxy-5-methylindol,
4-Hydroxy-1,5-dimethylindol,
4-Hydroxy-5-(dimethylaminomethyl)indol,
4-Hydroxy-5-methylindol,
2-Carboxymethyl-6-hydroxy-5-t-butylindol,
4-Hydroxy-7-methylindol,
5-Hydroxy-4-methylindol,
5-Hydroxy-6-methylindol,
6-Hydroxy-7-methylindol,
6-Hydroxy-7-(dimethylaminomethyl)indol,
5-Hydroxy-4-(dimethylaminomethyl)indol,
7-Hydroxy-6-(dimethylaminomethyl)indol,
5-Hydroxy-3,4-dimethylindol,
6-Hydroxy-3,7-dimethylindol,
4-Hydroxy-2,6-dimethylindol,
4-Hydroxy-6-hydroxymethylindol,
4-Hydroxy-6-methylindol und deren Salzen.

4. Verfahren gemäß jedem Anspruch 1 bis 3,
dadurch **gekennzeichnet**, daß
man auf die keratinische Materie eine Zusammensetzung (A) aufbringt, die, in einem zur Färbung geeigneten Milieu, mindestens ein substituiertes Monhydroxyindol der Formel (I) zusammen mit Jodidionen enthält, wobei die Aufbringung der Zusammensetzung (A) vor oder nach der Aufbringung einer Zusammensetzung (B) erfolgt, die, in einem zur Färbung geeigneten Milieu, Wasserstoffperoxid enthält.

5. Verfahren gemäß jedem Anspruch 1 bis 3,
dadurch **gekennzeichnet**, daß
man auf die keratinischen Fasern mindestens eine Zusammensetzung (A) aufbringt, die, in einem zur Färbung geeigneten Milieu, ein substituiertes Monohydroxyindol der Formel (I) zusammen mit Wasserstoffperoxid enthält und einen pH von 2 bis 7 aufweist, wobei die Aufbringung der Zuzsammensetzung (A) vor oder nach der Aufbringung einer Zusammensetzung (B) erfolgt, die, in einem zur Färbung geeigneten MIlieu, Jodidionen enthält.

6. Verfahren gemäß jedem Anspruch 4 oder 5,
dadurch **gekennzeichnet**, daß
die Jodionen in den Zusammensetzungen (A) oder (B) in Mengenverhältnissen von 0,007 bis 4 Gew.%, ausgedrückt als Ionen $J^-$, vorhanden sind, bezogen auf GEsamtgewicht der Zusammensetzung (A) oder (B).

7. Verfahren gemäß jedem Anspruch 1 bis 3,
dadurch **gekennzeichnet**, daß
man auf die keratinische Materie eine Zusammensetzung (A) auf Basis des substituierten Monohydroxyindols der Formel (I) und dann eine saure wässrige Zusammensetzung (B) aufbringt, wobei die Zusammensetzung (A) oder die Zusammensetzung (B) mindestens ein Nitrit enthalten, ausgewählt aus Alkali-, Erdalkali- oder Ammoniumnitriten oder aus Nitriten jedes weiteren kosmetisch verträglichen Kations, aus organischen Nitritderivaten und Vektoren von Nitriten, die ein Nitrit des oben definierten Typs erzeugen.

**8.** Verfahren gemäß Anspruch 7,
dadurch **gekennzeichnet**, daß
die Nitrite in Mengenverhältnissen von 0,02 bis 1 Mol/l vorhanden sind.

**9.** Verfahren gemäß jedem Anspruch 1 bis 3,
dadurch **gekennzeichnet**, daß
man auf die keratinischen Fasern eine Zusammensetzung (A), die, in einem zur Färbung geeigneten Milieu, ein substituiertes Monohydroxyindol der Formel (I) enthält, und vor oder nach der Zusammensetzung (A) eine Zusammensetzung (B) aufbringt, die, in einem zur Färbung geeigneten Milieu, ein Metallsalz von Mangan, Kobalt, Kupfer oder Silber enthält.

**10.** Verfahren gemäß Anspruch 9,
dadurch **gekennzeichnet**, daß
die Metallsalze in Mengenverhältnissen von 0,01 bis 2 Gew.%, ausgedrückt in Metallionen, verwendet werden, bezogen auf das Gesamtgewicht der Zusammensetzung.

**11.** Verfahren gemäß jedem Anspruch 1 bis 3,
dadurch **gekennzeichnet**, daß
man eine Zusammensetzung (A), die in einem zur Färbung geeigneten Milieu, mindestens ein substituiertes Monohydroxyindol, wie definiert in jedem der Ansprüche 1 bis 3, enthält, und vor oder nach dieser Zusammensetzung eine Zusammensetzung (B) aufbringt, die, in einem zur Färbung geeigneten Milieu, ein seltenes Erdmetallsalz enthält, ausgewählt aus den Salzen von Cer, Lanthan, Europium, Gadolinium, Ytterbium, Dysprosium.

**12.** Verfahren gemäß Anspruch 11,
dadurch **gekennzeichnet**, daß
die Salze der seltenen Erden in Mengenverhältnissen von 0,1 bis 8 Gew.%, bezogen auf Gesamtgewicht der Zusammensetzung, vorhanden sind.

**13.** Verfahren gemäß jedem der Ansprüche 1 bis 12,
dadurch **gekennzeichnet**, daß
bei Verwendung als Oxidationsmittel einer Zusammensetzung auf Basis von Wasserstoffperoxid der Gehalt an Wasserstoffperoxid in der Zusammensetzung im allgemeinen 1 bis 40 Volumina und vorzugsweise 2 bis 10 Volumina ausmacht.

**14.** Verfahren gemäß jedem Anspruch 1 bis 13,
dadurch **gekennzeichnet**, daß
die in der Zusammensetzung (A) verwndete Menge an substituierten Monohydroxyindolen der Formel (I) 0,01 bis 5 Gew.%, vorzugsweise 0,03 bis 2,5 Gew.%, bezogen auf Gesamtgewicht der Zusammensetzung, ausmacht.

**15.** Verfahren gemäß jedem Anspruch 1 bis 14,
dadurch **gekennzeichnet**, daß
die Zusammensetzungen (A) und (B) in Form von mehr oder weniger verdickten Lotionen, Cremes, Schäumen, Gelen vorliegen.

**16.** VErfahren gemäß jedem der Ansprüche 1 bis 15,
dadurch **gekennzeichnet**, daß
das zur Färbung geeignete MIlieu, das für die verschiedenen Zusammensetzungen (A) und/oder (B) herangezogen wird, ein wässriges Milieu aus Wasser oder einer Mischung aus Wasser und einem organischen Lösungsmittel ist oder auch durch ein wasserfreies Lösungsmittelmilieu dargestellt ist.

**17.** Verfahren gemäß Anspruch 16,
dadurch **gekennzeichnet**, daß
die verwendeten Lösungsmittel aus Ethyl-, Propyl- oder Isopropylalkohol, t-Butylalkohol, Ethylenglycol, Monomethyl-, Monoethyl- und Monobutylethern von Ethylenglycol, Monoethyletherethylenglycolacetat, Propylenglycol, Propylenglycol- und Dipropylenglycolmonomethylethern Methyllactat ausgewählt sind.

**18.** Zusammensetzung zur Verwendung bei der Färbung keratinischer fasern, dadurch **gekennzeichnet**, daß sie, in einem zur Färbung der keratinischen Fasern geeigneten Milieu, mindestens ein Monohydroxyindol der Formel (I):

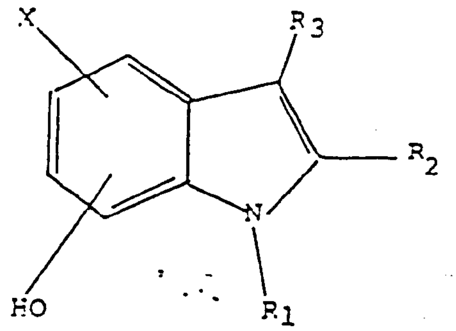

(I)

worin die OH-Gruppe eine jede Positionen des Benzolringes substituiert, X einen $C_{1-4}$-Alkylgruppe, der durch eine Hydroxygruppe substituiert ist oder nicht, wobei X auch Halogen oder eine Gruppe

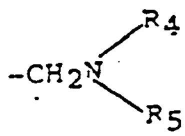

bedeuten kann, worin $R_4$ und $R_5$, unabhängig voneinander, einen $C_{1-4}$-Alkylrest oder Wasserstoff darstellen, $R_1$ ein Wasserstoffatom oder einen $C_{1-4}$-Alkylrest, $R_2$ und $R_3$, gleich oder verschieden, ein Wasserstoffatom, einen $C_{1-4}$-Alkylrest, einen Carboxyl- oder Alkoxicarbonylrest darstellen, sowie dessen Salze und mindestens Jodid- oder Nitritionen, enthält.

**19.** Mittel zur Färbung keratinischer FAsern, insbesondere menschlicher keratinischer Fasern, aus mehreren Bestandteilen, dadurch **gekennzeichnet**, daß es einen ersten Bestandteil aus einer Zusammensetzung (A), enthaltend ein substituiertes Monohydroxyindol der Formel (I), wie definiert in einem der vorhergehenden Ansprüche, und einen zweiten Bestandteil aus einer der Zusammensetzungen (B), wie definiert in einem der vorhergehenden Ansprücheumfaßt.

**20.** Vorrichtung aus mehreren Bestandteilen oder Kit zur Färbung, umfassend mehrere Teile:
- a) der erste Teil enthält eine Zusammensetzung (A), die ein substituiertes Monohydroxyindol der Formel (I), wie definiert in einem der vorhergehenden Ansprüche, ine inem wasserfreien Milieu enthält,
- b) ein zweiter Teil enthält eine Zusammensetzung (B), ausgewählt aus:
  - (i) Wasserstoffperoxid in einem zur Färbung keratinischer Fasern geeigneten Milieu,
  - (ii) einer Quelle von Jodidionen in einem zur Färbung der Fasern geeigneten Milieu,
  - (iii) einer Quelle von Nitriten in einer wässrigen Zusammensetzung mit einem sauren pH,
  - (iv) Metallsalzen, ausgewählt aus Salzen von Mangan, Kobalt, Kupfer und Silber, in einem zur Färbung der Fasern geeigneten Milieu,
  - (v) Salzen von seltenen Erden in einem zur Färbung von Fasern geeigneten Milieu; und
- c) ein dritter Teil ist dargestellt durch ein kosmetisch geeignetes wässriges Milieu, das dazu bestimmt ist, vor Gebrauch mit der im ersten Teil enthaltenen Zusammensetzung (A) vermischt zu werden.

## Claims

**1.** Process for dyeing keratinous fibres, in particular human keratinous fibres, characterised in that there is applied to these fibres a composition (A) containing, in a suitable dyeing medium, at least one substituted monohydroxyindole, of the formula:

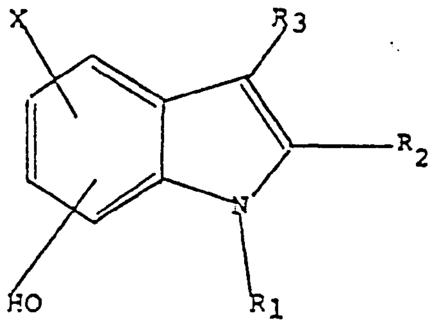

(I)

in which OH occupies any one of the positions on the benzene ring, X designates a $C_1$-$C_4$ alkyl radical, which may or may not be substituted by a hydroxyl group, it also being possible for X to designate halogen or a

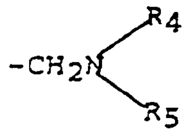

,

group, in which $R_4$ and $R_5$ designate a $C_1$-$C_4$ alkyl group, $R_1$ designates a hydrogen atom or a $C_1$-$C_4$ alkyl radical, $R_2$ and $R_3$, which may be identical or different, designate, independently of each other, a hydrogen atom, a $C_1$-$C_4$ alkyl radical, a carboxyl radical or an alkoxycarbonyl radical; as well as their salts, the colour being developed using an oxidising system consisting of:

(i) iodide ions and hydrogen peroxide, the composition (A) additionally containing in this case either (a) iodide ions or (b) hydrogen peroxide, the application of the composition (A) being preceded or followed by the application of a composition (B) which contains, in a suitable dyeing medium, either:

(a) hydrogen peroxide at a pH of between 2 and 12, when the composition (A) contains iodide ions, or

(b) iodide ions at a pH of between 3 and 11, when the composition (A) contains hydrogen peroxide;

(ii) nitrites, the application of the composition (A) being followed by the application of an aqueous composition (B) possessing an acid pH, the composition (A) or the composition (B) containing at least one nitrite;

(iii) metal salts chosen from among manganese, cobalt, copper and silver salts, these metal salts being applied in a separate stage by means of a composition (B) containing these salts in a suitable dyeing medium;

(iv) rare-earth metal salts, these rare-earth metal salts being applied by means of a composition (B) containing them in a suitable dyeing medium, the composition (B) being applied before or after the application of the composition (A).

2. Process according to Claim 1, characterised in that the application of the compositions (A) and (B) is separated by a rinsing stage with water.

3. Process according to either of Claims 1 or 2, characterised in that the monohydroxyindoles of formula (I) are chosen from among:

2-carboxy-4-hydroxy-5-methylindole,
4-hydroxy-1,5-dimethylindole,
4-hydroxy-5-(dimethylaminomethyl)indole,
4-hydroxy-5-methylindole,
2-carboxymethyl-6-hydroxy-5-tert-butylindole,
4-hydroxy-7-methylindole,
5-hydroxy-4-methylindole,
5-hydroxy-6-methylindole,
6-hydroxy-7-methylindole,
6-hydroxy-7-(dimethylaminomethyl)indole,
5-hydroxy-4-(dimethylaminomethyl)indole,

7-hydroxy-6-(dimethylaminomethyl)indole,
5-hydroxy-3,4-dimethylindole,
6-hydroxy-3,7-dimethylindole,
4-hydroxy-2,6-dimethylindole,
4-hydroxy-6-hydroxymethylindole,
4-hydroxy-6-methylindole;
and their salts.

**4.** Process according to any one of Claims 1 to 3, characterised in that there is applied to the keratinous materials a composition (A) containing, in a suitable dyeing medium, at least one substituted monohydroxyindole, of formula (I), in combination with iodide ions, the application of the composition (A) being preceded or followed by the application of a composition (B) which contains hydrogen peroxide in a suitable dyeing medium.

**5.** Process according to any one of Claims 1 to 3, characterised in that there is applied to the keratinous fibres at least one composition (A) containing, in a suitable dyeing medium, a substituted monohydroxyindole of formula (I), in combination with hydrogen peroxide and with a pH of between 2 and 7, the application of the composition (A) being preceded or followed by the application of a composition (B) which contains iodide ions in a suitable dyeing medium.

**6.** Process according to either of Claims 4 or 5, characterised in that the iodide ions are present in the compositions (A) or (B) in proportions of between 0.007 and 4% by weight expressed in I ions, relative to the total weight of the composition (A) or (B).

**7.** Process according to any one of Claims 1 to 3, characterised in that a composition (A) based on the substituted monohydroxyindole of formula (I) is applied to the keratinous materials and in that an acid aqueous composition (B) is then applied, the composition (A) or the composition (B) containing at least one nitrite chosen from among alkaline metal, alkaline-earth metal or ammonium nitrites or nitrites of any other cosmetically acceptable cation, organic derivatives both of nitrites and of nitrite vectors which generate a nitrite of the type defined above.

**8.** Process according to Claim 7, characterised in that the nitrites are present in proportions of between 0.02 and 1 mole/litre.

**9.** Process according to any one of Claims 1 to 3, characterised in that there is applied to the keratinous fibres a composition (A) containing, in a suitable dyeing medium, a substituted monohydroxyindole of formula (I) and in that there is applied before or after the composition (A), a composition (B) containing, in a suitable dyeing medium, a manganese, cobalt, copper or silver metal salt.

**10.** Process according to Claim 9, characterised in that the metal salts are used in proportions of between 0.01 and 2% by weight expressed in metal ions relative to the total weight of the composition.

**11.** Process according to any one of Claims 1 to 3, characterised in that there is applied a composition (A) containing, in a suitable dyeing medium, at least one substituted monohydroxyindole such as is defined in any one of Claims 1 to 3, and in that before or after this composition, there is applied a composition (B) containing, in a suitable dyeing medium, a rare-earth metal salt chosen from among cerium, lanthanum, europium, gadolinium, ytterbium and dysprosium salts.

**12.** Process according to Claim 11, characterised in that the rare-earth metal salts are present in proportions of between 0.1 and 8% by weight relative to the total weight of the composition.

**13.** Process according to any one of Claims 1 to 12, characterised in that when a composition based on hydrogen peroxide is used as oxidising means, the content of hydrogen peroxide in the composition is generally between 1 and 40 volumes and preferably between 2 and 10 volumes.

**14.** Process according to any one of Claims 1 to 13, characterised in that the amount of substituted monohydroxyindoles of formula (I) used in the composition (A) is between 0.01 and 5% by weight relative to the total weight of the composition, and preferably between 0.03 and 2.5% by weight.

**15.** Process according to any one of Claims 1 to 14, characterised in that the compositions (A) and (B) are

presented in the form of more or less thick lotions, creams, foams or gels.

16. Process according to any one of Claims 1 to 15, characterised in that the suitable dyeing medium used for the various compositions (A) and/or (B) is an aqueous medium consisting of water or a mixture of water and an organic solvent, or else consisting of an anhydrous solvent medium.

17. Process according to Claim 16, characterised in that the solvents used are chosen from among ethyl alcohol, propyl or isopropyl alcohol, tert-butyl alcohol, ethylene glycol, ethylene glycol monomethyl, monoethyl and monobutyl ethers, ethylene glycol monoethyl ether acetate, propylene glycol, propylene glycol and dipropylene glycol monomethyl ethers and methyl lactate.

18. Composition intended to be used in the dyeing of keratinous fibres, characterised in that it contains in a suitable medium for dyeing these keratinous fibres at least one monohydroxyindole of the formula (I):

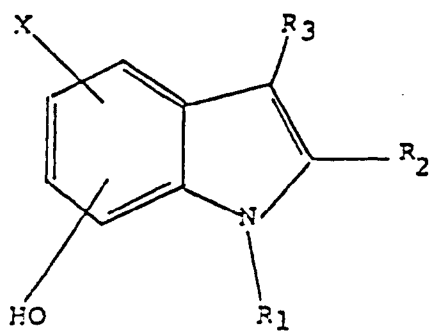

(I)

in which OH occupies any one of the positions on the benzene ring, X designates a $C_1$-$C_4$ alkyl radical, which may or may not be substituted by a hydroxyl group, it also being possible for X to designate halogen or a

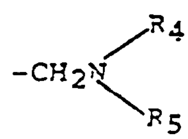

group, in which $R_4$ and $R_5$ designate,
independently of each other, a $C_1$-$C_4$ alkyl or hydrogen; $R_1$ designates a hydrogen atom or a $C_1$-$C_4$ alkyl radical, $R_2$ and $R_3$, which may be identical or different, designate a hydrogen atom, a $C_1$-$C_4$ alkyl radical, a carboxyl radical or an alkoxycarbonyl radical; as well as their salts, and at least iodide or nitrite ions.

19. Agent for dyeing keratinous fibres, in particular human keratinous fibres, containing a plurality of components, characterised in that it comprises a first component consisting of a composition (A), which contains a substituted monohydroxyindole of formula (I) such as is defined in one of the preceding claims, and a second component consisting of one of the compositions (B) such as is defined in one of the preceding claims.

20. Multi-compartment device or dyeing kit, comprising a plurality of compartments:
a) the first compartment containing a composition (A) containing a substituted monohydroxyindole of formula (I) such as is defined in one of the preceding claims in an anhydrous medium,
b) a second compartment containing a composition (B) chosen from:
(i) hydrogen peroxide in an acceptable medium for dyeing keratinous fibres,
(ii) a source of iodide ions in an acceptable medium for dyeing these fibres,
(iii) a source of nitrites in an aqueous composition having an acid pH,
(iv) metal salts chosen from manganese, cobalt, copper and silver salts in an acceptable medium for dyeing these fibres,
(v) rare-earth metal salts in an acceptable medium for dyeing the fibres; and
c) a third compartment consisting of a cosmetically acceptable aqueous medium, intended to be mixed with the composition (A) contained in the first compartment before use.